Europäisches Patentamt

European Patent Office

Office européen des brevets

(19)

(11) Veröffentlichungsnummer : **0 284 549 B1**

# (12) EUROPÄISCHE PATENTSCHRIFT

(45) Veröffentlichungstag der Patentschrift :
**15.07.92 Patentblatt 92/29**

(51) Int. Cl.$^5$ : **A61K 49/00,** A61K 49/02,
A61K 49/04, A61K 47/00

(21) Anmeldenummer : **88730073.9**

(22) Anmeldetag : **23.03.88**

(54) **Magnetische Flüssigkeitszusammensetzungen.**

(30) Priorität : **24.03.87 DE 3709851**

(43) Veröffentlichungstag der Anmeldung :
**28.09.88 Patentblatt 88/39**

(45) Bekanntmachung des Hinweises auf die
Patenterteilung :
**15.07.92 Patentblatt 92/29**

(84) Benannte Vertragsstaaten :
**AT BE CH DE ES FR GB IT LI LU NL SE**

(56) Entgegenhaltungen :
**EP-A- 0 071 564**
**EP-A- 0 077 752**
**EP-A- 0 124 766**
**EP-A- 0 184 849**
**EP-A- 0 186 616**

(56) Entgegenhaltungen :
**EP-A- 0 186 947**
**WO-A-85/05554**
**WO-A-86/01112**
**WO-A-87/02063**
**DE-A- 3 518 267**
**Radiology 147: 789-791, June 1983**

(73) Patentinhaber : **SILICA GEL GESELLSCHAFT
MBH ADSORPTIONS-TECHNIK,
APPARATEBAU
Ahornallee 36
W-1000 Berlin 19 (DE)**
Patentinhaber : **Pilgrimm, Herbert Dr.
Sophie-Charlotte-Str. 27a
W-1000 Berlin 37 (DE)**

(72) Erfinder : **Pilgrimm, Herbert, Dr.
Sophie-Charlotte-Str. 27a
W-1000 Berlin 37 (DE)**

EP 0 284 549 B1

**Beschreibung**

Die Erfindung betrifft magnetische Flüssigkeitzzusammensetzungen, die aus physiologisch verträglichen Dispersion von stabilisierten superparamagnetischen Teilchen in Wasser und einer für die Stabilisierung und für die chemische Bindung von diagnostischen und pharmakologisch wirksamen Substanzen ausreichenden Menge an reaktiven Stabilisatorsubstanzen, die mit der oberfläche der superparamagnetischen Teilchen über Phosphat- oder Phosphonatgruppen chemisch verbunden sind, bestehen.

Bei ihrem Einsatz als Kontrastmittel in der bildegebenden Diagnostik bewirken sie eine Erhöhung des Bildkontrastes und der Selektivität bei gleichzeitiger Verringerung der Konzentration gegenüber herkömmlichen Kontrastmitteln. Außerdem ergeben sich eine Vielzahl von neuen gewebespezifischen Kontrastmitteln und neuen Kontrastierungsverfahren sowie vielfältigen Einsatzmöglichkeiten in der Medizin und Veterinärmedizin.

Die Erhöhung des Bildkontrastes kann bei der Kernspintomographie von z.B. paramagnetischen Ionen, wie $Cr^{3\oplus}$, $Mn^{2\oplus}$, $Fe^{2\oplus}$, $Fe^{3\oplus}$, $CO^{2\oplus}$, $Ni^{2\oplus}$, $Cu^{2\oplus}$, $Gd^{2\oplus}$ oder stabile freie Radikale erreicht werden.

Aufgrund der geringen magnetischen Momente dieser Substanzen muß deren Konzentration relativ hoch sein, um eine wirksame Kontrastverbesserung zu erreichen. Da die meisten Substanzen in höherer Konzentration relativ toxish sind, kann bei den Ionen nur eine Komplexierung die Anwendbarkeit verbessern. So wurde in Radiology 147: 789-791, June 1983 ein paramagnetischer Chrom-EDTA-Komplex als mögliches intravenöses Kontrastmittel und Gadoliniumoxalat als orales Kontrastmittel für die Kernspintomographie vorgeschlagen. Der Einsatz des toxischen Gadoliniums für die intravenöse Kontrastmittelanwendung in der Kernspintomographie wird durch die Komplexierung mit organischen Komplexbildnern ermöglicht, wie in EP-A-0071564, DE-A-3401052, EP-A-0124766, WO 85/05554, EP-A-0184899 und EP-A-0186947 beschrieben wird. In WO 84/02642 werden u.a. Metallionen und Seltene-Erd-Metallionen enthaltene Porphyrine beschrieben.

Eine Vergrößerung der magnetischen Momente von Kontrastmitteln führte zum Einsatz größerer magnetischer Teilchen. So z.B. in der Patentanmeldung DE-A- 3590398, US-A- 4.675.173, US-A-4.615.879, WO 84/02643 und WO 85/04330. Hier werden ferromagnetische Mikroteilchen mit Weiß'schen Bezirken in der Größenordnung von einigen Hundert Ångströmeinheiten bis ca. 1 µm vorgeschlagen, die mit einer Polymerbeschichtung versehen werden, an die Substanzen mit Bindeaffinität für Gewebe gekoppelt werden.

Ferromagnetische Mikroteilchen in der vorgeschlagenen Größe haben so große magnetische Momente, daß die Teilchen sich zu größeren Aggregaten zusammenlagern, auch wenn sie mit einer Polymerbeschichtung versehen werden. Schon während der Beschichtung liegen die Teilchen aggregiert vor. Solche ferromagnetischen Mikroteilchen würden bei parenteraler Anwendung im Körper sedimentieren, ohne an den spezifischen Bindungsort zu gelangen. Die Verweildauer großer ferromagnetischer Mikroteilchen und deren Aggregate ist im Körper lang und die Gefahr von toxischen Nebenwirkungen groß.

Ähnliche Nachteile treffen auch für die in den Patentanmeldungen DE-A- 3443251 und DE-A- 3443252 verwendeten Dispersionen von ferromagnetischen Teilchen zu, wo die magnetischen Wechselwirkungen zwischen den Teilchen zu Aggregation und Sedimentation führen.

Die Magnetteilchen sind mit Mono-, Oligo- und Polysacchariden, Proteinen oder synthetischen Schutzkolloiden beschichtet, wobei die Bindung dieser Substanzen zu den Magnetteilchen über Adsorptionswechselwirkung erfolgt. Alle Faktoren, die die Struktur und die Zusammensetzung der Stabilisatormolekülschicht beeinflussen, können destabilisierend wirken, d.h. die Magnetteilchen können aggregieren und sedimentieren. Die vorgeschlagenen Stabilisatorsubstanzen liefern nur schwach stabilisierte Magnetteilchen, die z.T. sofort oder bei der Lagerung sedimentieren. Eine Anreicherung der Magnetpartikel im Organismus ist deshalb unspezifisch, die Gefahr von toxischen Nebenwirkungen groß.

Die Sedimentation der Magnetteilchen erfolgt besonders bei der Einwirkung von Magnetfeldern sehr schnell. Treten Magnetfeldinhomogenitäten auf, konzentrieren sich die Magnetteilchen immer an den Stellen höchster Feldstärke. Diese Nachteile treten besonders bei NMR-Diagnoseverfahren mit hohen Magnetfeldstärken auf.

In WO 85/02772 werden magnetische Teilchen mit nicht kovalent gebundenen Molekülen stabilisiert. Solche adsorptions-stabilisierten Magnetteilchen sind unter physiologischen Bedingungen nicht stabil, da durch Ablösung der Stabilisator-substanzen die Magnetteilchen leicht aggregieren. Werden an adsorptionsstabilisierten Magnetteilchen Substanzen mit Bindeaffinität für bestimmte Gewebe gekoppelt, so besteht die Gefahr, daß die Stabilisatorsubstanzen und somit die Substanzen mit Bindeaffinität von den kontrastgebenden Magnetteilchen abgelöst werden, die Magnetteilchen den Bindungsort nicht erreichen.

Die bisher hergestellten physiologisch verträglichen wäßrigen magnetischen Flüssigkeiten sind durch Adsorption von Stabilisatorsubstanzen erzeugt worden. Auch bei den Dextran-Magnetiten US-A- 4.101.435 und bei den Albumin-Magnetiten US-A- 4.452.773 erfolgt die Stabilisierung durch Adsorption großer Makromoleküle, die ähnliche Durchmesser wie die Magnetteilchen besitzen, so daß das wirksame Magnetfeld der Ma-

2

gnetteilchen auf benachbartes Gewebe gegenüber der Stabilisierung mit kleinen Molekülen stark verringert ist. Auch bei diesen magnetischen Flüssigkeiten tritt beim Verdünnen mit Blut eine Konkurrenzadsorption und Destabilisierung ein, die zur Aggregation der Magnetteilchen führen kann. Sie gelangen nicht zum gewünschten Bindungsort, wenn gewebespezifische Bindungssubstanzen gekoppelt sind.

Der in Anspruch 1 angegebenen Erfindung liegt die Aufgabe zugrunde, neue magnetische Flüssigkeitszusammensetzungen herzustellen, um die eingangs genannten Nachteile zu vermeiden und neue Anwendungsgebiete zu erschließen. Erfindungsgemäß wird das dadurch erreicht, daß sehr kleine superparamagnetische Eindomänenteilchen zur Anwendung kommen, die durch eine chemische Bindung von reaktiven Stabilisatorsubstanzen auf der Oberfläche der Magnetteilchen vor einer Aggregation geschützt werden.

Die superparamagnetischen Teilchen besitzen ein magnetisches Moment, das wesentlich größer ist als das von paramagnetischen Ionen, Radikalen oder Sauerstoff, und das annähernd den Maximalwert erreicht, der theoretisch für die jeweilige Materialzusammensetzung möglich ist. Die Magnetteilchen haben deshalb eine große Wirksamkeit auf die Verkürzung der Relaxationszeiten benachbarter resonanzfähiger Atomkerne und Moleküle, so daß die wirksamen Konzentrationen gegenüber den paramagnetischen Substanzen um Größenordnungen verringert werden können.

Es ist vorteilhaft, die superparamagnetischen Teilchen so klein als möglich herzustellen, um die biologische Halbwertszeit und die Toxizität möglichst gering zu halten, stabile magnetische Flüssigkeiten daraus herstellen zu können, die durch Filtration oder Hitzeeinwirkung sterilisierbar sind und die in sehr starken Magnetfeldern nicht aggregieren.

Erfindungsgemäß können sehr kleine superparamagnetische Teilchen durch Fällung aus einer gesättigten, organische Lösungsmittel enthaltenden Eisensalzlösung mit Laugen hergestellt werden.
Überraschend wurde gefunden, daß sich nahzu monodisperse superparamagnetische Teilchen sehr einfach herstellen lassen, indem zu einer Eisensalzlösung ein mit Wasser mischbares Lösungsmittel, wie Aceton, Ethylmethylketon, Dioxan, zugegeben wird, bis sich die erste beginnende Trübung durch Rühren gerade wieder auflöst. Aus solch einer Lösung können die superparamagnetischen Teilchen z.B. mit Natronlauge ausgefällt werden. Die Konzentration der Eisensalzlösung bestimmt die entstehende Teilchengröße. Je konzentrierter die Eisensalzlösung ist, desto kleiner werden die Teilchen.

Die Magnetteilchen liegen dabei im Durchmesserbereich von 1 bis 20 nm, vorzugsweise von 3-10 nm. Durch deren große spezifische Oberfläche (ca. 100 m$^2$/g) ist die Auflösungsgeschwindigkeit groß, d.h. die biologische Halbwertszeit im Organismus gering (1-3 Tage).

Als physiologisch verträgliche Magnetmaterialen kommen z.B. $\alpha$-Fe$_2$O$_3$, $\gamma$-Fe$_2$O$_3$, Fe$_3$O$_4$, Fe zur Anwendung, wobei Elemente wie Fluor oder Phosphor in physiologisch unbedenklichen Mengen in den Teilchen enthalten sein können. Dadurch kann die Diagnostik auch auf die Fluor-und Phosphor-NMR-Diagnostik ausgedehnt werden und durch Jodanteile die Kontrastverbesserung in der Röntgendiagiostik erhöht werden.

Erfindungsgemäß erfolgt die Stabilisierung der Magnetteilthei durch eine chemische Bindung von Stabilisatorsubstanzen auf der Oberfläche der Magnetteilchen. Die Stabilisatorsubstanzen müssen mindestens über eine chemisch reaktive funktionelle Gruppe, wie über eine Phosphat- oder Phosphonatgruppe verfügen, über die sie auf der Oberfläche der Magnetteilchen chemisch gebunden sind.

Der Molekülrest muß so beschaffe sein, daß er mit den verwendeten Dispersionsmitteln mischbar ist und den Magnetteilchenabstand so groß hält, daß die kinetische Energie der Magnetteilchen größer als die magnetische Wechselwirkungsenergie ist.

Überraschend wurde gefunden, daß organische wasserlösliche Mono-, Di-, Triphosphorsäureester, Mono-, Di-, Triphosphorsäureesterchloride, Mono- und Di-phosphate oder -phosphonate hydrolysestabile chemische Bindungen mit der Oberfläche der Magnetteilchen eingehen.

Für die erfindungsgemäßen wäßrigen magnetischen Flüssigkeitszusammensetzungen können die Stabilisatorsubstanzen z.B. aus Polyethylenglykolderivaten, wie Mono- und Di-polyethylenglykol-phosphat oder -phosphonat, Mono- und Di-polypropylenglykolpolyethylenglykol-phosphat oder -phosphonat, bestehen, wobei die Zahl der Propylenglykol -und Ethylenglykolgruppen zwischen 2 und 100 beträgt, aus $\omega$-Alkoxy-polyethylenglykol- und $\omega$-Alkoxy-polypropylenglykolpolyethylenglykol-phosphat oder -phosphonat bestehen, wobei die Zahl der Kohlenstoffatome der Alkoxygruppen zwischen 1 und 5 beträgt,
aus phosphatgruppenhaltigen Biomolekülen, wie Mono-, Di-, Triphosphorsäureester oder Mono-, Di-, Triphosphorsäureesterchloriden von Adenosin, Guanosin, Cytidin, Uridin, Thymidin, Desoxyadenosin. Desoxyguanosin, Desoxycytidin, Desoxythymidin, Inosin, Pyrimidin, Cytosin, Uracil, Thymin, Purin, Adenin, Guanin, Methylcytosin, 5-Hydroxymethylcytosin, 2-Methyladenin, 1-Methylguanin, Thiamin, Flavin, Riboflavin sowie Pyridoxalphosphat, Pyridoxaminphosphat, Ribonucleinsäuren, Desoxyribonucleinsäuren, Ribonucleinsäuresequenzen, Desoxyribonucleinsäuresequenzen, bestehen,
aus phosphatgruppenhaltigen Kohlehydraten bestehen, wobei die Kohlehydrate aus Monosacchariden, wie Glucose, Fructose, Ribose, Desoxyribose, Inosit,

aus Oligosacchariden, wie Saccharose, Raffinose, Gentianose, Malecitose, Stachyose, Verbascose,
aus Polysacchariden, wie Stärke, Lichenine, Glykogen, Dextrine, Dextrane, Inuline, Fruktosane, Lävane, Mannane, Galaktane, Xylane, Arabane, Pektine, Makropolysaccharide, Glycoproteide,
aus Polyglucuronsäure, Polygalacturonsäure, Polymannuronsäure, Alginsäure bestehen.

Erfindungsgemäß können mehrere Stabilisatorsubstanzen auf der Magnetteilchenoberfläche chemisch gebunden werden, DE-A- 3709852, wodurch die Dispergiereigenschaften in weiten Grenzen variierbar sind. So lassen sich z.B. stark polare Substanzen, wie Polyethylenglykolphosphat, und schwach polare Stabilisatorsubstanzen, wie Ölsäure, auf der Oberfläche der Magnetteilchen binden, so daß solche Teilchen in wäßrigem Blut transportiert und in fetthaltigem Gewebe angelagert werden können.

Die chemische Stabilisierung der Magnetteilchen erlaubt die Herstellung von magnetischen Flüssigkeitszusammensetzungen, die mit bekannten adsorptionsstabilisierten magnetischen Flüssigkeiten nicht möglich sind, da bei Konkurrenzadsorption die stärker adsorbierende Substanz die schwächer adsorbierte Substanz verdrängt.

Die so chemisch stabilisierten Magnetteilchen sedimentieren nicht, auch nicht unter der Wirkung von starken Magnetfeldern, was bei ihrem Einsatz in der NMR-Diagnostik sehr wichtig ist. Bei parenteraler Anwendung erfolgt keine Ablösung der Stabilisatorsubstanzen wie bei den bekannten adsorptionsstabilisierten Magnetteilchen, d.h, es erfolgt keine Aggregation und Sedimentation im Blut und damit eine gute Verteilung im Organismus. Erst wenn die chemisch gebundenen Stabilisatorsubstanzen abgebaut sind, kann eine Aggregation der Magnetteilchen erfolgen.

Erfindungsgemäß können an die mit der Magnetteichenoberfläche gebundenen Stabilisatorsubstanzen gewebespezifische Bindungssubstanzen gekoppelt werden, wenn die Stabilisatorsubstanzen noch freie reaktive chemische Gruppen besitzen, wie z.B. Hydroxyl-, Amin-, Aldehyd-, Epoxy-, Thiol-, Carboxyl -, 4,6-Dichlortriazin-, Hydroxamsäure-, Isocyanat-, Acylazid-, Anhydrid-, Diazoniumsalz-, Iminocarbonatgruppen.

Solche Stabilisatorsubstanzen sind z.B. Oligo- oder Polysaccharidphosphate, die vor oder nach der Bindung mit den Magnetteilchen mit den eitsprechenden funktionellen Gruppen versehen werden. Die Funktionalisierungstechniken sind aus den Immobilisierungsverfahren der Biochemie bekannt und Stand der Technik. Außer diesen reaktiven Stabilisierungs-substanzen auf Kohlhydratbasis sind noch funktionalisierte Polyethylenglykole, oder Polyethylenglykolpolypropylenglykole, wie ω-Oxoalkoxy-polyethylenglykol-phosphat oder -phosphonat oder deren Acetale, ω-Aminoalkoxy-polyethylenglykol -phosphat oder -phosphonat, ω-Oxoalkylamino-polyethylenglykol-phosphat oder -phosphonat oder deren Acetale, ω-Oxoalkoxy-polyethylenglykolpolypropylenglykol-phosphat oder -phosphonat oder deren Acetale, ω-Oxoalkylamino-polyethylenglykolpolypropylenglykol-phosphat oder -phosphonat oder deren Acetale,
oder reaktive phosphatgruppenhaltige Biomoleküle, wie z.B. Pyridoxalphosphat, Pyridoxaminphosphat oder Cocarboxylase als reaktive Stabilisatorsubstanzen anwendbar.

Erfindungsgemäß können an die mit der Magnetteilchenoberfläche gebundenen reaktiven Stabilisatorsubstanzen nach den in der Biochemie bekannten Immobilisierungsverfahren die gewebespezifischen Bindungssubstanzen, wie z.B. Antigene, Antikörper, Haptene, Ribonucleinsäuren, Desoxyribonucleinsäuren, Protein A, Hormone, Hormonanaloge, gekoppelt werden.

Die so über die Stabilisierungssubstanzen chemisch mit den Magnetteilchen verbundenen gewebespezifischen Bindungssubstanzen bilden magnetfeldstabile Flüssigkeiten, die nicht agglomerieren und sich deshalb im Körper gut verteilen und an den Bindungsorten anreichern. Es erfolgt auch keine Desorption der Stabilisator- oder Bindungssubstanzen in die Körperflüssigkeit. Erst mit dem enzymatischen Abbau der Substanzen erfolgt die Destabilisierung der Magnetteilchen.

Erfindungsgemäß können neben den gewebespezifischen Bindungssubstanzen auch pharmakologisch wirksame Substanzen, wie z.B. Antitumorproteine, Antitumorenzyme, Antibiotika, Pflanzenalkaloide, Alkylierungsreagenzien, Lymphokine, Gewebe-Plasminogen-Aktivatoren, Urocinase, Lymphotoxine, Makrophagen-Aktivierungskörper, Tumor-Nekrose-Faktoren, an die freien reaktiven Gruppen der Stabilisatorsubstanzen gekoppelt werden, so daß neben den gewebespezifischen Anlagerungen der Magnetteilchen auch eine pharmakologische Wirkung am Bindungsort erzielt werden kann.

Die Toxizität solcher pharmakologisch wirksamer Substanzen kann dabei relativ hoch sein, da die Magnetteilchen sich aufgrund ihrer gewebespezifischen Wechselwirkung bevorzugt an den entsprechenden Bindungsorten anreichern und dort ihre pharmakologische Wirkung, z.B. zur Tumorbekämpfung, ausüben. Die Dosis der pharmakologisch wirksamen Substanz kann gering gehalten werden, da sich die Substanz am Wirkungsort konzentriert und der übrige Körper nur gering belastet wird.

Bei einigen Substanzen, wie z.B. bei den Gewebe-Plasminogen-Aktivatoren, Urocinase, sind die gewebespezifisthen Bindungseigenschaften und die pharmakologischen Wirkungseigenschaften gekoppelt, so daß nur diese Substanzen an die Stabilisierungssubstanzen gebunden zu werden brauchen, um eine spezifische Anlagerung am Blutgerinsel und dessen Auflösung zu bewirken.

4

Die Kopplung pharmakologisch wirksamer Substanzen an Magnetteilchen hat weiterhin den Vorteil, daß über die Relaxationszeitverkürzung am Wirkungsort der Therapieerfolg mit der in vivo-NMR-Diagnostik beobachtet werden kann.

Erfindungsgemäß können an die mit der Magnetteilchenoberfläche gebundenen reaktiven Stabilisatorsubstanzen pharmakologisch wirksame biologisch aktive Teilchen, wie Organellen, Viren, Mikroben, Algen, Pilze, Zellen und hier insbesondere Erythrozyten, Leukozyten, Thrombozyten, Langerhans'sche Inseln, gekoppelt werden.

Diese von einer wenige nm dicken Schicht von Magnetteilchen umgebenen biologischen Teilchen können als NMR- und Ultraschallkontrastmittel angewendet werden.

Ein weiteres Anwendungsgebiet dieser biologisch aktiven magnetischen Teilchen ist die Erzeugung von physiologisch wichtigen Stoffwechselprodukten im Körper oder die Entfernung von toxischen Stoffen aus dem Körper durch die Fixierung der magnetischen biologisch aktiven Teilchen im Blutkreislauf oder Körperhöhlen durch magnetische Hochgradientenfelder. So lassen sich z.B. magnetische Langerhans'sche Inseln durch starke inhomogene Magnetfelder in Körperhöhlen fixieren und zur Insulinproduktion bei Diabetes einsetzen.

Erfindungsgemäß können an die mit der Magnetteilchenoberfläche gebundenen reaktiven Stabilisatorsubstanzen pharmakologisch wirksame Komplexbildner, wie Polycarbonsäuren, Aminocarbonsäuren, Porphyrine, Katecholamine, gebunden werden. Solche magnetischen Flüssigkeitszusammensetzungen können zur Bindung von toxischen Substanzen im Körper eingesetzt werden, die mit den magnetischen Komplexbildnern eine Verbindung eingehen. Die entstehenden magnetischen Komplexe lassen sich mit Hilfe von magnetischen Hochgradientenfeldern abtrennen.

So kann z.B. der Blutkreislauf durch eine außerhalb des Körpers befindliche Schlauchleitung geführt werden und kontinuierlich eine magnetische Flüssigkeitszusammensetzung in den Blutkreislauf gegeben werden, die die darin enthaltenen toxischen Bestandteile oder auch andere Blutbestandteile, wie Viren, Bakterien, Krebszellen, magnetisiert und im magnetischen Hochgradientenfeld vom Blut abtrennt.

Erfindungsgemäß können an die mit der Magnetteilchenoberfläche gebundenen reaktiven Stabilisatorsubstanzen jodhaltige reaktive Substanzen, wie z.B. 2,4,6-Trijod-benzoesäure, 2,4,6-Trijod-isophthalsäure, 2,4,6-Trijod-5-aminoisophthalsäure, 2,4,6-Trijod-phenylessigsäure, 2,4,6-Trijod-phenylpropionsäure und deren Derivate, 3,5-Dijod-tyrosin, L-Thyroxin chemisch gebunden werden.

Die so gebildeten Röntgenkontrastmittel haben gegenüber den bisherigen jodhaltigen Kontrastmiteln verbesserte Eigenschaften, da die örtliche Volumenkonzentration der absorptionsfähigen Eisen- und Jodatome sehr hoch ist. Man benötigt weniger Kontrastmittel, die Verträglichkeit der magnetischen Kontrastmittel ist besser, die Kontrastmittel diffundieren nicht in den Extrazellularraum.

Erfindungsgemäß kann zur Kontrasterhöhung bei der NMR-Diagnostik eine Kombination von positiv kontrastbildenden paramagnetischen Metallkomplexen oder freien Nitroxidradikalen und negativ kontrastbildenden magnetischen Flüssigkeitszusammensetzungen eingesetzt werden, wobei die einzelnen Kontrastmittel zeitlich nacheinander oder in Form von Mischungen gleichzeitig verabreicht werden können. Die in den Extrazellularraum diffundierenden paramagnetischen Metallkomplexe bilden in der Gefäßwand einen positiven NMR-Kontrast und die nicht durch die Gefäßwand diffundierenden magnetischen Flüssigkeitszusammensetzungen liefern einen negativen NMR-Kontrast.

Erfindungsgemäß können in den Magnetteilchen und in den Stabilisatorsubstanzen NMR-aktive Elemente, wie Phosphor und Fluor, enthalten sein, so daß neben der Protonenresonanz auch die Phosphor- und Fluorresonanz sowie deren relaxationszeitverändernde Wirkung für die NMR-Diagnostik anwendbar wird.

So lassen sich bei der Herstellung der superparamagnetischen Teilchen durch Fällung Fluor- und/oder Phosphoratome, wie z.B. als Fluorid-, Phosphat- oder Hexafluorophosphationen, in die Magnetteilchen einbringen. Damit liefern die Magnetteilchen Fluor- und/oder Phosphor-NMR-Signale, die für diagnostische Zwecke ausgenutzt werden können.

Ähnliche Wirkungen lassen sich erreichen, indem fluor- und/oder phosphorhaltige Substanzen neben den obigen genannten Stabilisatorsubstanzen chemisch mit der Oberfläche der Magnetteilchen verbunden werden. Solche Stabilisatorsubstanzen sind z.B. Perfluoralkoxy-polyethylenglykol-phosphat, -carbonsäuren, -phosphonate, -sulfonamidphosphate, -sulfonamidphosphonate, Perfluoralkylcarbonsäuren, -phosphate, -phosphonate, -sulfonamidphosphate, -sulfonamidphosphonate, -sulfonamidcarbonsäuren, Perfluorpolyethylen-glykol-phosphat, -phosphonat, Phytinsäure oder phosphatgruppenhaltige Kohlehydrate, die fluorhaltige Substituenten, wie z.B. Perfluoralkyl-, Perfluoralkoxypolyethylenglykol-, Perfluorakylsulfonamidgruppen, enthalten.

Erfindungsgemäß können jodhaltige Röntgenkontrastmittel, die Phosphat-, Phosphonat oder Carboxylgruppen enthalten, neben den oben genannten Stabilisatorsubstanzen chemisch mit der Oberfläche der Magnetteilchen verbunden werden, so daß Röntgeikontrastmittel mit hoher Absorptionsfähigkeit für Röntgenstrahlung entstehen. Solche jodhaltigen Kontrastmittel sind, wie schon oben aigegeben, z.B. 2,4,6-Trijod-benzoesäure und deren Derivate.

Die Herstellung der magnetischen Flüssigkeitszusammensetzung erfolgt durch Mischen der superparamagnetischen Teilchen mit den Stabilisierungssubstanzen und dem Dispersionsmittel und anschließender thermischer Reaktion der reaktiven funktionellen Gruppen der Stabilisatorsubstanzen mit den Hydroxyl-gruppen auf der Oberfläche der Magnetteilchen.

Die superparamagnetischen Teilchen lassen sich durch mechanisches Zerkleinern größerer Magnetteilchen, durch thermische Zersetzung von Eisencarbonylen und nachfolgender gezielter Umsetzung mit Wasserdampf oder durch Fällung von Eisensalzen und entsprechender Oxidation oder Reduktion der Niederschläge herstellen.

An einem Beispiel soll die erfindungsgemäße Herstellung von Magnetit-Pulvern erläutert werden.

Beispiel 1:

270 g Eisen(III)-chlorid und 99 g Eisen(II)-Chlorid werden in 500 ml Wasser gelöst. Durch Zugabe von Aceton wird die Eisensalzlösung bis zur beginnenden Trübung in eine gesättigte Eisensalzlösung überführt. Durch Zugabe von Natronlauge oder Ammoniakwasser wird der pH -Wert auf 10,5 gebracht und die Mischung auf 70°C erwärmt. Nach dem Abkühlei wird der entstehende Niederschlag abfiltriert oder abzentrifugiert und mit Wasser chloridfrei gewaschen. Die entstehenden Magnetitpartikel können entweder sofort weiterverarbeitet oder schonend bei niedriger Temperatur im Vakuum getrocknet werden:

Aus diesen Magnetitteilchen kann durch 2-stündiges Einleiten eines Luft-Wasserdampf-Gemisches bei 100°C superparamagnetisches $\alpha$-$Fe_2O_3$ hergestellt werden.

Erhitzt man die Magnetitteilchen in einem Autoklaven in einer Luft-Wasserdampf-Atmosphäre auf 250°C, bilden sich superparamagnetische $\gamma$-$Fe_2O_3$-Teilchen.

Die Stabilisierung der Magnetteilchen erfolgt bei Raumtemperatur, gegebenenfalls bei erhöhter Temperatur.

Beispiel 2:

10 g Magnetit-Pulver werden mit 3 g $\omega$-Methoxy-decaethylenglykol-phosphat und 100 ml Wasser gemischt und 2 Stunden im Autoklav bei 140°C gerührt. Die entstehende sterile wäßrige magnetische Flüssigkeit wird durch ein 0,2 $\mu$m Filter filtriert und die überschüssige Stabilisierungssubstanz durch Dialyse entfernt. Die entstehende magnetische Flüssigkeit wird mit Natriumchlorid zu einer 0,9-%-igen Kochsalzdispersion gemischt. Nach Verdünnung mit physiologischer Kochsalzlösung auf die gewünschte Endkonzentration kann die NMR-diagnostische magnetische Flüssigkeitszusammensetzung abgefüllt werden.

Bei der Stabilisierung der Magnetpartikel mit zwei oder mehreren Stabilisatorsubstanzen ist das gewünschte stöchiometrische Verhältnis zu beachten, das die Dispersionseigenschaften der entstehenden stabilisierten Magnetpartikel bestimmt. So ist ein $\gamma$-$Fe_2O_3$-Pulver, das mit 3 Teilen Methoxy-deca-ethylenglykol-phosphat und 1 Teil Ölsäure gemischt wird, weder in Wasser noch in Benzin gut mischbar. Ein geeignetes Dispersionsmittel ist aber z.B. n-Butylacetat.

Beispiel 3:

10 g $\gamma$-$Fe_2O_3$ werden mit 2,3 g Methoxy-deca-ethylenglykol-phosphat, 0,8 g Ölsäure und 100 ml n-Butylatetat gemischt und 4 Stunden in einer Apparatur mit Wasserabscheider unter Rückfluß gekocht. Die entstehende stabile magnetische Flüssigkeit wird filtriert und das n-Butylacetat im Vakuum abgedampft. Es entsteht ein Rückstand, der in physiologischer Kochsalzlösung löslich ist und als NMR-diagnostische Flüssigkeitszusammensetzung verwendbar ist.

Die Stabilisierung der Magnetpartikel mit Stabilisatorsubstanzen, die für eine Bindung von gewebespezifischen oder pharmakologisch wirksamen Substanzen geeignet sind, kann wie im Beispiel 4 erläutert erfolgen. Die anschließende Kopplung der gewebespezifischen Bindungssubstanzen und der pharmokologisch wirksamen Subztanzen wird je nach den zur Verfügung stehenden reaktiven Gruppen nach dem Stand der Technik durchgeführt.

Beispiel 4:

10 g Magnetit-Pulver nach Beispiel 1 werden mit 3,5 g Polygalacturonsäurephosphat und 100 ml Wasser gemischt und 2 Stunden im Autoklav bei 140°C gerührt. Die entstehende magnetische Flüssigkeit wird durch ein 0,2 $\mu$m Filter filtriert und gegen Wasser dialysiert. Zu 10 ml dieser magnetischen Flüssigkeit (20 mg stabilisiertes Magnetit/ml magnetische Flüssigkeit) werden 10 mM Natriumperjodat gegeben und nach 2-stündiger Oxidation gegen eine kalte 0,02 M Natriumborat-Pufferlösung von pH 8,5 dialysiert.

Die entstehenden Magnetitteilchen besitzen an den chemisch gebundenen Polygalacturonmolekülen Aldehydgruppen, die z.B. mit Aminogruppen von gewebespezifischen Bindungssubstanzen und/oder pharmakologisch wirksamen Substanzen zur Reaktion gebracht werden können. Die sich aus diesen Reaktionen bildenden Schiff'schen Basen werden anschließend mit Natriumborhydrid stabilisiert.

Beispiel 5:

1 ml mit Natriumperjodat oxidierte magnetische Flüssigkeit nach Beispiel 4 wird mit 1 ml Human-$\gamma$-Globulin (1 mg/ml) verrührt und nach 12 Stunden durch Zugabe von 1 ml 0,2M Natriumborhydridlösung 60 Minuten reduziert. Die entstehende magnetische Flüssigkeit wird gegen physiologisthe Kochsalzlösung dialysiert und enthält chemisch gebundenes $\gamma$-Globulin auf mit Polygalacturonsäurephosphat stabilisierten Magnetteilchen. Diese magnetische Flüssigkeitszusammensetzung ist für die NMR-Diagnostik anwendbar.

Beispiel 6:

10 g Magnetit-Pulver nach Beispiel 1 werden mit 4 g $\omega$-Oxoalkylamino-polyethylenglykol-phosphat mit 20 Ethylenglykoleinheiten in 20 ml Wasser gemischt und 30 min, im Ultraschallbad bei 80°C dispergiert. Die entstehende reaktive magnetische Flüssigkeit wird mit 2 g 2,4,6-Tri-jod-5-aminoisophthalsäure gemischt und nach 20 min. gegen eine physiologische Kochsalzlösung dialysiert. Nach der Filtration durch ein 0,2 $\mu$m-Filter ist die entstehende magnetische Flüssigkeitszusammensetzung als Röntgenkontrastmittel verwendbar.

Beispiel 7:

10 g Magnetit-pulver nach Beispiel 1 werden mit 4 g $\omega$-Oxoalkoxy-dekapolyethylenglykol-phosphonat und mit 20 ml physiologischer Kochsalzlösung gemischt und 30 min. im Ultraschallbad bei 80°C dispergiert. Die entstehende reaktive magnetische Flüssigkeit wird mit 5 g Erythrozyten versetzt und 30 min. bei Raumtemperatur stehen gelassen. Die entstehende magnetische Flüssigkeitszusammensetzung wird mit 4 g Harnstoff versetzt und nach 10 min. gegen physiologische Kochsalzlösung dialysiert.

Die entstehenden magnetischen Flüssigkeitszusammensetzungen können zur Ultraschalldiagnostik eingesetzt werden.

Die erfindungsgemäßen magnetischen Flüssigkeitszusammensetzungen können sowohl für die Kernspintomographie als auch für die lokale magnetische Kernresonanz -Spektroskopie Anwendung finden. Beim Einsatz fluorhaltiger magnetischer Flüssigkeitszusammensetzungen bietet sich die Möglichkeit der leichten Identifizierung im Körper an, da die mittlere Konzentration der Fluoratome im Körper sehr niedrig ist.

Neben dieser wichtigen in vivo-Anwendung können die erfindungsgemäßen magnetischen Flüssigkeitszusammensetzungen auch für die in vitro-Diagnostik Anwendung finden, so z.B. als magnetische DNA-Sonde. Hier werden DNA-Sequenzen, die für bestimmte pathogene DNA spezifisch sind, isoliert und an magnetische Teilchen gebunden. Diese DNA-sequenzhaltigen magnetischen Flüssigkeiten werden nun zu klinischen Proben gegeben; in denen die Viren oder Bakterien DNA einzelsträngig freigelegt wurden. Es erfolgt eine Hybridisierung, wenn die gesuchte pathogene DNA in der Probe enthalten ist. Die Probe kann nun NMR-Spektroskopisch untersucht oder die Hybridisierungsprodukte im magnetischen Hochgradientenfeld isoliert, gewaschen und analysiert werden.

Nach diesem Prinzip lassen sich die magnetischen Flüssigkeitszusammensetzung mit gewebespezifischen Bindungssubstanzen für vielfältige Anwendungen in der in vitro-Diagnostik einsetzen.

Die erfindungsgemäßen magnetischen Flüssigkeitszusammensetzungen werden im Körper, insbesondere bei der Stabilisierung mit Polyethylenglykol-phosphat oder Ethoxy-polyethylenglykol-phosphat, zu Eisen abgebaut, das sich zum größten Teil in den vom Körper gebildeten Erythrozyten wiederfindet. Sie können deshalb als Eisenpräparat bei Eisenmangelerkrankungen Anwendung finden.

Die erfindungsgemäßen magnetischen Flüssigkeitszusammensetzungen werden bei der in vivo-Anwendung zum großen Teil in der Leber gespeichert und dort abgebaut, so daß cytostatisch wirksame magnetische Flüssigkeitszusammensetzungen, die z.B. als Stabilisatorsubstanzen $\omega$-Oxoalkoxy-polyethylenglykolphosphat oder -phosphonat, $\omega$-Oxoalkylamino-polyethylenglykol-phosphat oder -phosphonat oder deren Acetale enthalten, auch ohne gewebespezifische Bindungssubstanzen für die Therapie von Lebertumoren einsetzbar sind.

Die erfindungsgemäßen magnetischen Flüssigkeitszusammensetzungen können auch als magnetische Carrier für den Transport pharmakologisch wirksamer Substanzen verwendet werden, wobei durch Einwirkung von inhomogenen Magnetfeldern die Anreicherung der Magnetteilchen in bestimmten Gewebe- und Körperbereichen unterstützt wird.

Die erfindungsgemäßen magnetischen Flüssigkeitszusammensetzungen lassen sich auch vielseitig in der Technik einsetzen, so z.B. bei der Trennung von Feststoffen unterschiedlicher Dichte im Magnetfeld, zur Magnetisierung von Bakterien, Viren, Zellen usw. in der Biotechnologie und der biologischen Abwasserreinigung, als Absorptionsflüssigkeit bei magnetischen Stofftrennverfahren, als Absorptionsflüssigkeit für Sonnenstrahlung.

## Patentansprüche

1. Physiologisch verträgliche superparamagnetische Flüssigkeitszusammensetzungen, **gekennzeichnet durch** einen Gehalt an einer Dispersion aus feinverteilten superparamagnetischen Eisenoxid- oder Eisenteilchen in Wasser und einen Gehalt an organischen Stabilisatorsubstanzen, bestehend aus phosphat- oder phosphonatgruppenhaltigen Polyethylenglykolen, phosphatgruppenhaltigen Nucleotiden, deren Oligomeren oder deren Polymeren, phosphatgruppenhaltigen Kohlehydraten, die über diese Gruppe an die Oberfläche der Magnetteilchen chemisch gebunden sind und gegebenenfalls über diese Gruppen einen chemisch gebundenen Gehalt an diagnostisch oder pharmakologisch wirksamen Substanzanteil aufweisen.

2. Physiologisch verträgliche superparamagnetische Flüssigkeitszusammensetzungen nach Anspruch 1, **gekennzeichnet dadurch**, daß die superparamagnetischen Teilchen aus $\alpha$-Fe$_2$O$_3$, $\gamma$-Fe$_2$O$_3$, Fe$_3$O$_4$ oder Eisen bestehen, die Eisenoxid-Teilchen einen bis zu 10 Gew.% Phosphor- und/oder Fluoranteil enthalten können und der Teilchendurchmesser zwischen 1 und 20 nm, insbesondere zwischen 3 und 10 nm liegt.

3. Physiologisch verträgliche superparamagnetische Flüssigkeitszusammensetzungen nach Anspruch 1, **gekennzeichnet dadurch**, daß die Stabilisatorsubstanzen aus polyethylenglykolhaltigen Substanzen Mono- und Dipolyethylenglykol-phosphat oder -phosphonat, aus $\omega$-Alkoxy-polyethylenglykol-phosphat oder -phosphonat, aus Mono- und Dipolypropylenglykolpolyethylenglykol-phosphat oder -phosphonat, aus $\omega$-Alkoxy-polypropylenglykolpolyethylenglykol-phosphat oder -phosphonat bestehen, bei denen die Zahl der Kohlenstoffatome der Alkoxygruppen zwischen 1 und 5 und die Zahl der Ethylenglykolgruppen zwischen 2 und 100 beträgt,

aus den phosphatgruppenhaltigen Nucleotiden Mono-, Di-, Tri-phosphorsäureester oder Mono-, Di-, Triphosphorsäureesterchloriden von Adenosin, Guanosin, Cytidin, Uridin, Thymidin, Desoxyadenosin, Desoxyguanosin, Desoxycytidin, Desoxythymidin, Inosin, Pyrimidin, Cytosin, Uracil, Thymin, Purin, Adenin, Guanin, Methylcytosin, 5-Hydroxymethyl-cytosin, 2-Methyladenin, 1-Methylguanin, Thiamin, Flavin, Riboflavin sowie Pyridoxalphosphat, Pyridoxaminphosphat, Ribonucleinsäuresequenzen, Desoxyribonucleinsäuresequenzen, Ribonucleinsäure, Desoxyribonücleinsäuren bestehen;

aus phosphatgruppenhaltigen Kohlenydraten bestehen, wobei die Kohlenhydratreste aus den Monosacchariden Glucose, Fructose, Ribose, Desoxyribose, Inosit, den Oligosacchariden Saccharose, Raffinose, Gentianose, Malecitose, Stachyose, Verbascose,

den Polysacchariden Stärke, Lichenine, Glykogen, Dextrine, Dextrane, Inuline, Fruktosane, Lävane, Mannane, Galaktane, Xylane, Arabane, Pektine, Makropolysaccharide, Glycoproteide, Polyglucuronsäure, Polygalacturonsäure, Polymannuronsäure, Alginsäure bestehen.

4. Physiologisch verträgliche superparamagnetische Flüssigkeitszusammensetzungen, nach Anspruch 1, **gekennzeichnet dadurch**, daß die für die Stabilisierung und für die chemische Bindung von diagnostisch und pharmakologisch wirksamen Substanzen zu verwendenden reaktiven Stabilisatorsubstanzen über mindestens zwei chemisch reaktive funktionelle Gruppen verfügen, wobei die Phosphat- bzw. Phosphonatgruppe für die chemische Bindung zu den superparamagnetischen Teilchen dient und die restlichen reaktiven funktionellen Gruppen, die aus Hydroxyl-, Amin-, Aldehyd-, Oxiran-, Thiol-, 4,6-Dichlortriazin-, Hydroxamsäure-, Isocyanat-, Acylazid-, Anhydrid-, Diazoniumsalz- oder Iminocarbonatgruppen bestehen für die Bindung des diagnostisch oder pharmakologisch wirksamen Substanzanteils dienen und daß die reaktiven Stabilisatorsubstanzen aus $\omega$-Oxoalkoxy-polyethylenglykol-phosphat oder -phosphonat oder deren Acetale, $\omega$-Oxoalkoxy-polyethylenglykol-polypropylen-glykolphosphat oder -phosphonat oder deren Acetale, $\omega$-Oxoalkylamino-polyehtylenglykol-phosphat oder -phosphonat oder deren Acetale,

$\omega$-Oxoalkylamino-polyethylenglykol-polypropylenglykol-phosphat oder -phosphonat oder deren Acetale bestehen, aus $\omega$-Aminoalkoxy-polyethylenglykol-phosphat oder -phosphonat, $\omega$-Aminoalkylamino-polyethylenglykol-phosphat oder -phosphonat bestehen, wobei die Zahl der Kohlenstoffatome der Alkylgruppen zwischen 1 und 5 und die Zahl der Ethylenglykolgruppen zwischen 2 und 100 und die Zahl der Propylenglykolgruppen zwischen 2 und 100 beträgt, aus phosphatgruppenhaltigen Kohlehydraten gemäß Anspruch 3 bestehen und außerdem noch die restlichen reaktiven funktionellen Gruppen, die Aldehyd-, Amin-, Oxiran-, 4,6-Dichlortriazin-, Isocyanat-, Acylazid-, Anhydrid-, Diazoniumsalz- oder Iminocarbonatgruppe bedeuten.

5. Physiologisch verträgliche superparamagnetische Flüssigkeitszusammensetzungen nach den Ansprüchen 1, 3 und 4,

**gekennzeichnet dadurch**, daß neben den Stabilisierungssubstanzen gemäß den Ansprüchen 1, 3 und 4 noch die fluorhaltigen Substanzen Perfluoralkylphosphate, -phosphonate, -sulfonamidphosphate, -sulfonamidphosphonate, ω-Perfluoralkoxy-polyethylenglykolphosphate, -phosphonate, -carbonsäuren, wobei die Zahl der Kohlenstoffatome der Perfluoralkylgruppen zwischen 2 und 8 beträgt, Perfluorpolyethylenglykol-phosphate, -phosphonate und/oder den jodhaltigen Substanzen 2,4,6-Trijod-benzoesäure, 2,4,6-Trijod-isophthalsäure, 2,4,6-Trijod-5-aminoisophthalsäure, 2,4,6-Trijod-phenylessigsäure, 2,4,6-Trijod-phenylpropionsäure, 3,5-Dijodtyrosin, L-Thyroxin und deren Derivate, die ebenfalls an die Oberfläche der superparamagnetischen Teilchen chemisch gebunden sind, enthalten sind.

6. Physiologisch verträgliche superparamagnetische Flüssigkeitszusammensetzungen nach den Ansprüchen 1, 3 und 4

**gekennzeichnet dadurch**, daß an die mit der Magnetteilchenoberfläche gebundenen Stabilisatorsubstanzen die jodhaltigen Substanzen 2,4,6-Trijod-benzoesäure, 2,4,6-Trijod-isophthalsäuresäure, 2,4,6-Trijod-5-aminoisophthalsäure, 2,4,6-Trijod-phenyl-essigsäure, 2,4,6-Trijod-phenylpropionsäure, 3,5-Dijod-tyrosin, L-Thyroxin und deren Derivate in bekannter Weise chemisch gebunden sind.

7. Physiologisch verträgliche superparamagnetische Flüssigkeitszusammensetzungen nach den Ansprüchen 1, 3-6

**gekennzeichnet dadurch**, daß gewebespezifische Bindungssubstanzen aus Antigenen, Antikörpern, Ribonucleinsäuren, Desoxyribonucleinsäuren, Ribonucleinsäuresequenzen, Desoxyribonucleinsäuresequenzen, Haptenen, Protein A, Hormonen, Hormonanalogen, aus den pharmakologisch wirksamen Substanzen Antitumorproteinen, Enzymen, Antitumorenzymen, Antibiotikas, Pflanzenalkaloiden, Alkylierungsreagenzien, Lymphokeinen, Lymphotoxinen, Urokinase, Gewebe-Plasminogen-Aktivatoren, Makrophagen-Aktivierungskörpern, Tumor-Nekrose-Faktoren, aus den pharmakologisch wirksamen Organellen, Viren, Mikroben, Algen, Pilzen, Zellen, insbesondere Erythrozyten, Leukozyten, Langerhans'schen Inseln oder den pharmakologisch wirksamen komplexbildenden Polycarbonsäuren, Aminocarbonsäuren, Porphyrinen oder Katecholaminen in bekannter Weise an die Stabilisatorsubstanzen chemisch gebunden sind.

8. Verfahren zur Herstellung physiologisch verträglicher superparamagnetischer Flüssigkeitszusammensetzungen gemäß den Ansprüchen 1-7

**gekennzeichnet dadurch**, daß die superparamagnetischen Eisenoxidteilchen aus einer gesättigten, mit Wasser mischbaren organische Lösungsmittel enthaltenden wäßrigen Eisensalzlösung mit Alkalilauge oder Ammoniakwasser ausgefällt, mit 30 bis 100 Gew.% Stabilisatorsubstanz versetzt und unter erhöhter Temperatur und gegebenenfalls erhöhtem Druck zur Reaktion gebracht und in an sich bekannter Weise gereinigt und gegebenenfalls noch mit diagnostisch oder pharmakologisch wirksamen Substanzen in an sich bekannter Weise gekoppelt werden.

9. Verwendung von physiologisch verträglichen superparamagnetischen Flüssigkeitszusammensetzungen gemäß den Ansprüchen 1-7 für die enterale und parenterale bildgebende NMR-, Röntgen-, Ultraschall-Diagnostik,

**gekennzeichnet dadurch**, daß man entweder die physiologisch verträglichen superparamagnetischen Flüssigkeitszusammensetzungen allein oder in Kombination mit paramagnetischen Metallkomplexen oder freien Nitroxidradikalen zeitlich nacheinander oder in Form von Gemischen gleichzeitig verabreicht.

10. Physiologisch verträgliche superparamagnetische Flüssigkeitszusammensetzungen gemäß den Ansprüchen 1-7 zur Entfernung von im Blutkreislauf enthaltenen Viren, Bakterien, Zellen oder toxischen Stoffen aus dem Körper.

## Claims

1. Physiologically tolerated superparamagnetic liquid compositions, comprise by a content of dispersion of finely dispersed superparamagnetic iron oxid or iron particles in water and an amount of organic stabilizer substances, which comprise phosphate or phosphonate groups containing polyethyleneglycols, phosphate groups containing nucleotids, their oligomers or polymers, phosphate groups containing carbohydrates, which over these groups are chemically bonded to the surface of the magnetic particles and if necessary over these groups have a chemically bonded content of diagnostic and pharmacologically active substances.

2. Physiologically tolerated superparamagnetic liquid compositions of claim 1, wherein the superparamagnetic particles are selected from the group consisting of $\alpha$-Fe$_2$O$_3$, $\gamma$-Fe$_2$O$_3$, Fe$_3$O$_4$ and iron, the iron oxide particles contain up to 10 % by weight of phosphorus and/or fluorine and the particle diameter is between 1 and

EP 0 284 549 B1

10 nm and especially between 3 and 10 nm.

3. Physiologically tolerated superparamagnetic liquid compositions of claim 1, wherein the stabilizer substances comprise polyethyleneglycol groups containing substances mono- and di-polyethyleneglycol phosphate or phosphonate, ω-alkoxy-polyethyleneglycol phosphate or phosphonate, mono- and di-polypropyleneglycol-polyethyleneglycol phosphate or phosphonate ω-alkoxy-polypropyleneglycol-polyethyleneglycol phosphate or phosphonate, the number of carbon atoms of the alkoxy groups is between 1 and 5 and the number of ethyleneglycol groups is between 2 and 100,

phosphate groups containing nucleotides mono-, di- and triphosphate esters and mono-, di- and triphosphoric acid chlorides of adenosine, guanosine, cytidine, uridine, thymidine, desoxyadenosine, desoxyguanosine, desoxycytidine, desoxythymidine, inosine, pyrimidine, cytosine, uracil, thymine, purine, adenine, guanine, methylcytosine, 5-hydroxy-methylcytosine, 2-methyladenine, 1-methylguanine, thiamine, flavine, riboflavine, and pyridoxal phosphate, pyridoxamine phosphate, ribonucleic acids, desoxyribonucleic acids, ribonucleic acid sequences, desoxyribonucleic acid sequences, orotidylic acid;

phosphate groups containing carbohydrates, the carbohydrate groups comprising the monosaccharides, glucose, fructose, ribose, desoxyribose, inositol;

the oligosaccharides sucrose, raffinose, gentianose, malecitose, stachyose, verbascose;

the polysaccharides starch, lichenins, glycogen, dextrins, dextrans, inulins, fructosans, levans, mannans, galactans, xylans, arabans, pectins,

macropolysaccharides, glycoproteids, polyglucoronic acid, polycalacturonic acid, polymannuronic acid, alginic acid.

4. Physiologically tolerated superparamagnetic liquid compositions of claim 1, wherein the reactive stabilizer substances used for the stabilisation and chemical bonding of diagnostic and pharmacologically effective substances, have at least two chemically reactive functional groups, one of the functional groups being phosphate or phosphonate for chemical bonding to the superparamagnetic particles and the other reactive functional groups, such as hydroxyl, amine, aldehyde, oxiran, thiol, 4,6-dichlorotriazine, hydroxamic acid, isocyanate, acylazide, anhydride, diazonium salt or iminocarbonate groups serving to bond diagnostic and pharmagolocically active substances and wherein the reactive stabilizer substances comprise group consisting of ω-oxoalkoxy-polyethyleneglycol phosphate or phosphonate or their acetals, ω-oxoalkoxy-polyethyleneglycol-polypropyleneglycol phosphate or phosphonate or their acetals, ω-oxoalkylamino-polyethyleneglycol phosphate or phosphonate or their acetals, ω-alkylamino-polyethyleneglycol phosphate or phosphonate or their acetals, ω-oxoalkylamino-polyethyleneglycol-polypropyleneglycol phosphate or phosphonate or their acetals, ω-aminoalkoxy-polyethyleneglycol phosphate or phosphonate, ω-aminoalkylaminopolyethyleneglycol phosphate or phosphonate, the number of carbon atoms of the alkyl groups being between 1 and 5, the number of ethyleneglycol groups between 2 and 100 and the number of propyleneglycol groups being between 2 and 100, phosphate groups containing carbohydrates of claim 3 and, moreover, containing the reactive functional groups aldehyde, amine, oxirane, 4,6-di-chlortriazine, isocyanate, acylazide, anhydride, diazonium salt and iminocarbonate groups.

5. Physiologically tolerated superparamagnetic liquid compositions of claims 1, 3 and 4, wherein aside from the stabilizer substances of claims 3 and 4, there are also contained fluorine-containing substances perfluoroalkyl phosphates, phosphonates, sulfonamide phosphates, sulfonamide phosphonates, ω-perfluoroalkoxypolyethyleneglycol phosphates, phosphonates, carboxylic acids, the number of carbon atoms of the perfluoroalkyl groups being between 2 and 8, perfluoropolyethyleneglycol phosphates or phosphonates, and/or iodine-containing reactive substances 2,4,6-triiodo-benzoic acid, 2,4,6-triiodo-isophthalic acid, 2,4,6-triiodo-5-aminoisophthalic acid, 2, 4, 6-triiodo-phenylacetic acid, 2, 4, 6-triiodo-phenylpropionic acid and their derivatives, 3, 5-diiodo-tyrosine and L-thyroxine which are also bonded chemically to the surface of the superparamagnetic particles.

6. Physiologically tolerated superparamagnetic liquid compositions of claims 1, 3 and 4, wherein the stabilizer substances, which are bonded chemically to the surface of the magnetic particles, comprise iodine-containing substances 2, 4, 6-triiodo-benzoic acid, 2, 4, 6-triiodo-isophthalic acid, 2,4,6-triiodo-5-aminoisophthalic acid, 2,4,6-triiodo-phenylacetic acid, 2,4,6-triiodo-phenylpropionic acid, 3,5-diiodo-tyrosine, L-thyroxine and their derivatives.

7. Physiologically tolerated superparamagnetic liquid compositions of claims 1, 3 to 6, wherein tissue-specific bonding substances antigens, antibodies, ribonucleic acids, desoxyribonucleic acids, ribonucleic acid sequences, desoxy-ribonucleic acid sequences, haptens, protein A, hormones, hormone analogs, pharmacologically active substances antitumor proteins, enzymes, antitumor enzymes, antibiotics, plant alkaloids, alkylating reagents, lymphokines, lymphotoxins, urokinase, tissue plasminogen activators, macrophage activators, tumor necrosis factors, pharmacologically effective organelles, viruses, microbes, algae, fungi, cells, especially erythrocytes, leukocytes, Langerhans'islets, pharmacologically active complex-

10

ing agents polycarboxylic acids, aminocarboxylic acids, porphyrins, catecholamines, are chemically bonded to the stabilizer substances.

8. A method for the preparation of magnetic liquid compositions of claims 1 to 7, wherein the superparamagnetic iron oxid particles are prepared by precipitation from a saturated, with water miscible organic solvent-containing iron salt solution with alkaline or ammonia solutions, 30 to 100 % by weight of stabilizer substances added, and by increased temperature, and if necessary increased pressure, brought to reaction, cleaned, and if necessary with diagnostic and pharmacologically active substances chemically bonded.

9. Use of physiologically tolerated superparamagnetic liquid compositions of claims 1 to 7 for enteral and parenteral image-forming NMR, radiological and ultrasonic diagnosis, wherein the superparamagnetic liquid compositions being administered alone or consecutively or simultaneously in the form of mixtures in combination with paramagnetic metal complexes or free nitroxide radicals.

10. Physiologically tolerated superparamagnetic liquid compositions of claims 1 to 7 to remove viruses, bacteria, cells or toxic materials from the circulating blood of the body.

## Revendications

1. Composés liquides superparamagnétiques tolérés par l'organisme,
**caractérisés par** une composante issue d'une dispersion dans l'eau de particules superparamagnétiques d'oxyde de fer ou de fer finement réparties, d'une composante issue de substances stabilisatrices organiques composées de polyéthylène-glycoles contenant un groupe phosphaté ou phosphonaté, de nucléotides contenant un groupe phosphaté ainsi que de leurs oligomères ou de leurs polymères, d'hydrates de carbone contenant un groupe phosphaté permettant leur liaison chimique aux particules magnétiques, et éventuellement par une composante issue de substances diagnostiques ou à effet pharmacologique fixées chimiquement grâce à ces groupes.

2. Composés liquides superparamagnétiques tolérés par l'organisme conformes à la demande 1,
**caractérisés par** des particules superparamagné-tiques composées de $\alpha$-Fe$_2$O$_3$, de $\gamma$-Fe$_2$O$_3$, de Fe$_2$O$_4$ ou de fer, par des particules d'oxyde de fer composées de phosphore et/ou de fluor pour une part pouvant aller jusqu'à 10% de leur poids, et par des particules dont le diamètre est compris entre 1 et 20 nm et plus particulièrement entre 3 et 10 nm.

3. Composés liquides superparamagnétiques tolérés par l'organisme conformes à la demande 1,
**caractérisé**s par des substances stabilisatrices composées notamment des substances suivantes contenant du polyéthylèneglycole phosphate ou phosphonate de mono ou de dipolyéthylèneglycole et sont également composées de
phosphates ou de phosphonates, $\omega$-alcoxy-polyéthylenèglycole phosphates ou phosphonates, mono ou de dipolypropylèneglycolepolyéthylèneglycole phosphates ou phosphonates, $\omega$-alcoxy-polypropylèneglycolepolyéthylèneglycole sur lesquels le nombre d'atomes de carbone des groupes alcoxyliques est compris entre 1 et 5 et celui des groupes d'éthylèneglycole entre 2 et 100,
de nucléotides contenant des groupes phosphatés, mono, di, ou triesters d'acidephosphorique ou mono, di, ou trichlorides d'ester d'acide phosphorique d'adénosine, de guanosine, de cytidine, d'uridine, de thymidine, de désoxyadénosine, de désoxyguanosine, de désoxycytidine, de
désoxythymidine, d'inosine, de pyrimidine, de cytosine, d'uracile, de thymine, de purine, adénine, de guanine, de cytosine de méthyle, de cytosine d'hydroxyméthyle 5, d'adénine de méthyle 2, de guanine de méthyle 1, de thiamine, de flavine, de riboflavine ainsi que de phosphate pyridoxal, de phosphate pyridoxaminique, de séquences d'acides ribonucléiques, de séquences d'acides désoxyribonucléiques, d'acides ribonucléiques, d'acides désoxyribonucléiques;
contenant également des hydrates de carbone comprenant des groupes phosphatés et sur lesquels les restes d'hydrates de carbone sont composés des monosaccharides suivants glucose, fructose, ribose, désoxyribose, inosite,
des oligosaccharides suivants saccharose, raffinose, gentianose, malécitose, stachyose, verbascose, des polysaccharides suivants amidon, lichenine, glycogène, dextrine, dextrane, inuline, fructosane, lévane, mannane, galactane, xylane, arabane, pectine,
macropolysaccharide, glycoprotéide, acide polyglucuronique, acide polygalacturonique, acide polymannuronique, acide alginique.

4. Composés liquides superparamagnétiques tolérés par l'organisme conformes à la demande 1,
**caractérisés par** l'utilisation, pour la stabilisation et la fixation chimique de substances diagnostiques ou à effet pharmacologique, de substances stabilisatrices réactivés comportant au moins deux groupes fonctionnels réagissant chimiquement le groupe phosphaté ou phosphonaté permet la liaison chimique avec les particules superparamagnétiques, tandis que les autres groupes fonctionnels réactifs, composés d'hydroxyles, d'amines, d'aldéhydes, d'oxiranes, de thioles, de dichlortriazine 4 et 6, d'acide hydroxamique, d'isocyanates, d'acylazides, d'anhydrides, de sel diazoniomique, ou d'iminocarbonates assurent la fixation de la substance diagnostique ou a effet pharmacologique, mais ils sont également caractérisés par des substances stabilisatrices réactives composées de phosphate ou de phosphonate ω-oxoalcoxy-polyéthylèneglycole ou de leurs acétals, de phosphate ou de phosphonate ω-oxoalcoxy-polyéthylèneglycole- polypropylène-glycole ou de leurs acétals, de phosphate ou de phosphonate ω-oxoalcylamino-polyéthylèneglycole ou de leurs acétals, de phosphate ou de phosphonate ω-oxoalcylamino-polyéthylèneglycolepolypropylèneglycole ou de leurs acétals, ainsi que de phosphate ou de phosphonate ω-aminoalcoxypolyéthylèneglycole et de phosphate ou de phosphonate ω-aminoalcylamino-polyéthylèneglycole pour lesquels le nombre des atomes de carbone des groupes alcyliques est compris entre 1 et 5, celui des groupes éthylèneglycole entre 2 et 100 et celui des groupes propylèneglycole entre 2 et 100, d'hydrates de carbone contenant des groupes phosphatés conformément à la demande 3 et enfin ils sont caractérisés par les autres groupes réactifs fonctionnels, c'est à dire le groupe aldéhyde, amine, oxirane, dichlortriazine 4 ou 6, isocyanate, acylazide, anhydride, sel azionomique ou iminocarbonate.

5. Composés liquides superparamagnétiques tolérés par l'organisme conformes aux demandes 1, 3, et 4, **caractérisés**, en plus des substances stabilisatrices conformément aux demandes 1, 3, et 4, par les substances fluorées suivantes phosphates, phosphonates, sulfonamidphosphates et sulphonamidphosphonates perfluoralcyliques, phosphates, phosphonates et acides carboniques ω-perfluoralcoxy-polyéthylèneglycole pour lesquels le nombre d'atomes de carbone des groupes perfluoralcyliques est compris entre 2 et 8, phosphates, phosphonates et/ou substances contenant de l'iode , en l'occurrence acide benzoïque triiodique 2, 4, et 6, acide isophthalique triiodique 2, 4, et 6, acide aminoisophthalique 5 triiodique 2, 4, 6, acide phénylacétique triiodique 2, 4, 6, acide phénylepropionique triiodé 2, 4, 6, tyrosine triodée 3 et 5, thyroxine L ainsi que leurs dérivés qui sont également fixés chimiquement à la surface des particules superparamagnétiques perfluorpolyéthylèneglycole.

6. Composés liquides superparamagnétiques tolérés par l'organisme conformes aux demandes 1, 3, et 4, **caractérisés par** des substances contenant de l'iode, en l'occurrence acide benzoïnique triiodique 2, 4, 6, acide isophthalique triiodique 2, 4, 6, acide aminoisophtalique 5 triiodique 2, 4, 6, acide phénylacétique triiodique 2, 4, 6, acide phénylepropionique triiodique 2, 4, 6, tyrosine diiodique 3 et 5, thyroxine L ainsi que leurs dérivés dont on connaît le type de liaison chimique avec les substances stabilisatrices elles-mêmes fixées à la surface des particules magnétiques.

7. Composés liquides superparamagnétiques tolérés par l'organisme conformes aux demandes 1, et 3 à 6, **caractérisés par** des substances de liaison spécifiques aux tissus, composées d'antigènes, d'anticorps, d'acides ribonucléiques, d'acides désoxyribonucléiques, de séquences d'acides ribonucléiques, de séquences d'acides désoxyribonucléiques, d'haptènes, de protéines A, d'hormones, d'hormonalogènes, composées également de substances à effet pharmacologique, en l'occurrence protéines antitumeurs, enzymes, enzymes antitumeurs, antibiotiques, alcaloïdes végétaux, lymphocéines, lymphotoxines, urocinase, activateurs plasminogènes de tissus, corpuscules d'activation des macrophages, agents de nécrose des tumeurs, composées enfin d'organites, de virus, de microbes, d'algues, de champignons, de cellules, en l'occurrence erytrocytes, leucocytes, îlots de Langerhans en particulier, à effet pharmacologique ou d'acides polycarbonés, d'acides aminocarbonés, de porphyrines ou de catécholamines complexes à effet pharmacologique, et dont on connaît le type de liaison chimique aux substances stabilisatrices.

8. Procédé de fabrication de composés liquides superparamagnétiques tolérés par l'organisme conformes aux demandes 1 à 7, **caractérisés par** les opérations suivantes précipitation des particules d'oxyde de fer superparamagnétiques, issues d'une solution aqueuse de sel de fer saturé contenant un solvant organique alliable à de l'eau, avec de la lessive alcaline ou de l'eau ammoniacale, mélange avec une substance stabilisatrice dans une proportion comprise entre 30 et 100% de leur poids et réaction à température et éventuellement à pression élevées, purification suivant un procédé déjà connu et éventuellement couplage suivant un procédé déjà connu avec des substances diagnostiques ou à effet pharmacologique.

9. Utilisation de composés liquides superparamagnétiques tolérés par l'organisme conformes aux demandes 1 à 7 pour les procédés de diagnostique par images entérales ou parentérales NMR, Röntgen, par ultrasons, **caractérisée** soit par une utilisation de ces composés liquides seuls, soit par une utilisation de ces composés en association avec des complexes métalliques paramagnétiques ou des radicaux libres nitroxidiques administrés successivement, ou simultanément sous forme de mélanges. 10. Composés liquides superparamagnétiques tolérés par l'organisme conformes aux demandes 1 à 7 pour l'élimination de virus, de bactéries, de cellules ou de substances toxiques contenus par l'appareil circulatoire.